(19) Europäisches Patentamt
European Patent Office
Office européen des brevets

(11) Publication number: 0 327 379 A2

(12) # EUROPEAN PATENT APPLICATION

(21) Application number: 89301059.5

(22) Date of filing: 03.02.89

(51) Int. Cl.4: C 07 C 103/54
C 07 C 103/70,
C 07 D 207/404, A 61 K 7/00,
C 07 C 103/44

(30) Priority: 05.02.88 GB 8802646

(43) Date of publication of application:
09.08.89 Bulletin 89/32

(84) Designated Contracting States:
BE DE FR GB IT NL SE

(71) Applicant: CRODA INTERNATIONAL plc
Cowick Hall Snaith
Goole North Humberside DN14 9AA (GB)

(72) Inventor: Coupland, Keith
2 White Cottages South Cliffe
Hotham York YO4 3UX (GB)

Smith, Peter John
86 Etherington Road
Beverley Road Hull HU6 73P (GB)

(74) Representative: Wain, Christopher Paul et al
A.A. THORNTON & CO. Northumberland House 303-306
High Holborn
London WC1V 7LE (GB)

(54) **Humectants.**

(57) Compounds of the formulae:

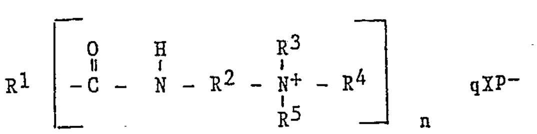

(I)

or

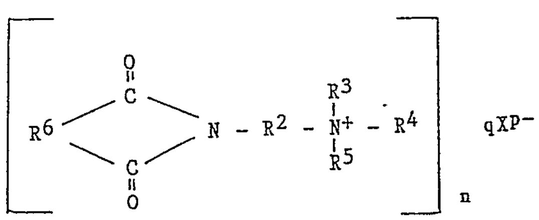

(II)

are useful as humectants e.g. in cosmetic compositions. In the formulae, n is 1 or 2, q and p are positive integers and the product of q and p is equal to n; $R^1$ is a saturated or unsaturated hydrocarbyl radical and when n is 1, $R^1$ can be hydrogen, and when n is 2, $R^1$ can be a single bond; $R^2$ is a substituted or unsubstituted alkylene group; $R^3$, $R^4$ and $R^5$ are substituted and unsubstituted alkyl groups; and $R^6$ is ethylene, substituted ethylene, or an ortho-aromatic structure. The compounds are prepared by reacting a carboxylic acid or derivative thereof with an N, N-disubstituted alkyldiamine in order to form a corresponding amide, and then quaternizing the amide with an alkylating agent.

EP 0 327 379 A2

## Description

### HUMECTANTS

The present invention relates to certain compounds which are useful as humectants, and to their use in cosmetic compositions. The invention also relates to methods for preparing these compounds and compositions.

Various compounds are known to be useful as humectants. These compounds include certain modified proteins and quaternized acetamides and lactamides. British patent specification No. 1,554,251 describes cosmetic compositions which contain, as humectants, one or more N-hydroxyalkyl alkane acid amides such as, for example N-(2-hydroxyethyl) - acetamide.

We have now discovered certain further compounds which are useful as humectants, particularly in cosmetic compositions. These new humectants not only absorb moisture, but also adhere well to the skin and hair. Humectants of the present invention have the formula:

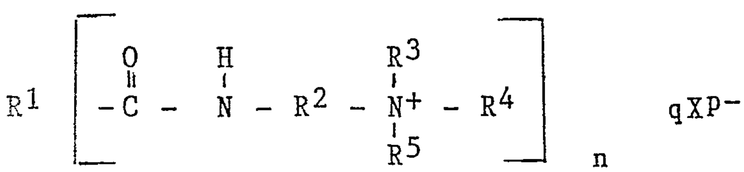

(I)

or

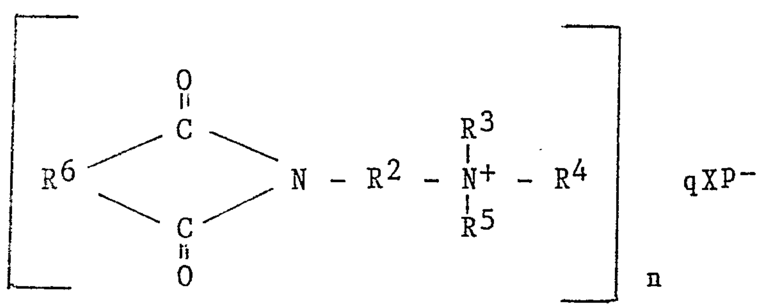

(II)

in which n is 1 or 2, and q and p are positive integers, with the product of q and p being equal to n; and wherein $R^1$ is a saturated or unsaturated hydrocarbyl radical, and where n is 1, $R^1$ may also be hydrogen, and when n is 2, $R^1$ may also be a single bond; and wherein $R^2$ is a substituted or unsubstituted alkylene group, and $R^3$, $R^4$ and $R^5$ are independently selected from substituted and unsubstituted alkyl groups; and wherein $R^6$ is an ethylene group, a substituted ethylene group, or an ortho-aromatic compound.

In one preferred embodiment of the present invention, $R^1$ is a saturated or unsaturated aliphatic hydrocarbon group which can optionally include one or more substituents, such as hydroxy groups. In accordance with another embodiment of the present invention, $R^2$ can be a substituted or unsubstituted propylene group, and preferably $R^3$, $R^4$ and $R^5$ are a substituted or unsubstituted methyl group.

The compounds of the present invention can be prepared by reacting a carboxylic acid or carboxylic acid derivative such as an ester, with an N, N-disubstituted alkyldiamine, in order to form the corresponding amide, and then quaternizing the amide with an alkylating agent. In accordance with a preferred embodiment, the N, N-disubstituted alkyldiamine has the formula

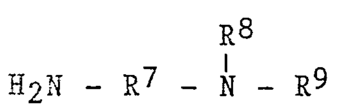

(III)

in which $R^7$ is a substituted or unsubstituted alkylene group, and $R^8$ and $R^9$ are independently selected from substituted and unsubstituted alkyl groups.

The carboxylic acids and derivatives thereof employed can be saturated or unsaturated, and mono-, di-, or polybasic. They may optionally also contain substituents, such as hydroxy groups. When a monobasic or mono-carboxylic acid is employed, the compound of formula I is obtained in which n is 1. Examples of these mono-carboxylic acids include formic acid, acetic acid, propionic acid, lactic acid, butyric acid, pentanoic acid, undecanoic acid, dodecanoic acid, tridecanoic acid, tetradecanoic acid, pentadecanoic acid, hexadecanoic acid, heptadecanoic acid, octadecanoic acid, nonadecanoic acid, eicosanoic acid, docosanoic acid, tetracosanoic acid, and mixtures thereof. The preferred monobasic acids are formic, propionic, and lactic acid.

In the case where a polybasic or polycarboxylic acid is utilized (other than succinic acid) a compound of formula I is obtained in which n is 2 or more. These polybasic acids include, for example, oxalic acid, malonic acid, glutaric acid, adipic acid, pimelic acid, sebacic acid, and mixtures thereof. When oxalic acid is used, for example, $R^1$ is a single bond in formula I. When succinic acid (or a substituted derivative thereof) is used, a compound of formula II is produced. As indicated above, the carboxylic acid can also be unsaturated, i.e. it can be a monobasic unsaturated acid such as acrylic acid, octadecenoic acid, docosenoic acid, and the like, or it can be an unsaturated di-carboxylic acid such as fumaric acid.

The humectant compounds of this invention can also be produced from mixtures of acids, such as the acids from naturally occurring oils, such as the coconut oil fatty acids. When a mixture of acids is used, a corresponding mixture of the compounds of formula I and/or II is, of course, formed. Other acids are, for example, caprylic (octanoic) acid, oleic (cis-9-octodecanoic) acid, and behenic (docosanoic) acid.

The N,N-disubstituted alkyldiamines which are reacted with the carboxylic acids or derivatives thereof in accordance with the method of this invention are preferably of the formula

$$H_2N - R^7 - \underset{\substack{|\\ R^8}}{N} - R^9 \qquad (III)$$

in which divalent group $R^7$ is a substituted or unsubstituted alkylene group, most preferably a propylene group. $R^7$ can have from about 2 to 10 carbon atoms, and can include one or more substitutents, provided that these do not adversely affect the desired humectant properties of the final product. The N-substituents, $R^8$ and $R^9$, are substituted or unsubstituted alkyl groups, preferably methyl. In general, lower alkyl groups are preferred. As noted, the $R^8$ and $R^9$ groups can contain substituents, such as hydroxyl groups.

The amide formed by reaction between the carboxylic acid or derivative thereof and the N, N-disubstituted alkyldiamine is quaternized with an alkylating agent, generally designated $R^{10}pX$. The $R^{10}$ group is preferably an alkyl group, more preferably a lower alkyl group, and most preferably a methyl group. It can also contain one or more substituents, such as hydroxyl groups. The anion X is preferably a halide, particularly a chloride ion, but other mono- or multivalent anions can also be used. It is preferred, however, not to use dimethyl sulfate as the alkylating agent.

The humectant compounds of the present invention, i.e. those of formulae I and II, are useful inter alia in cosmetic compositions. Thus, in particular, these humectants can be employed in cosmetic, toiletry or household compositions, preferably containing between about 1 and 25 wt.% of the humectant hereof. The humectants are particularly useful in cosmetic products for the care or protection of skin or hair, or in toiletry products for cleansing or conditioning skin or hair. The humectants can also be used in other household products, for example for the cleansing, conditioning or care of various surfaces, textiles and articles. When employed in these various cosmetic compositions, the humectants are preferably used in combination with other substances such as emulsifiers, lipids, vegetable extracts, thickeners, surfactants, solvents, perfumes, colouring agents, and the like.

In order that the invention may be more fully understood, the following Examples are given by way of illustration only.

EXAMPLE 1

408 grams of dimethylaminopropylamine (4 moles) were added to a 2 litre flask equipped with a stirrer, nitrogen sparge, thermometer, temperature controller and splash head take-off. 252 grams of acetic acid (4.2 moles) were then slowly added, with the whole being purged with nitrogen. The reaction was initially exothermic, reaching 110°C, and then the mixture was heated to 150°C, while maintaining the nitrogen sparge, in order to drive off the water of reaction and the slight excess of acetic acid. After four hours at 150°C, the mixture was then allowed to cool, to yield a pale yellow liquid. The liquid analysed as:

| | |
|---|---|
| Acid Value | 3.0 (mg KOH/gram) |
| Base Value | 379 (mg KOH/gram) |
| Theoretical Base Value | 383 (mg KOH/gram) |

The conversion was 97.3% based on the base value. Infrared spectra were consistent with the structure: $CH_3$-CO-NH-$(CH_2)_3$-N(Me)$_2$

EXAMPLE 2

Quaternization

70 grams of the product of Example 1, along with 70 grams of methanol, were added to a 250 ml glass flask fitted with a gas inlet, thermometer and reflux condenser. This mixture was then brought to reflux (at 68°C) and methyl chloride gas were passed therethrough for three hours. After cooling, the methanol was removed on a rotary film evaporator under vacuum at 60°C. This yielded a white crystalline solid with a base value of 3.2 (i.e. 99.2% conversion to quaternary). The product was shown to be very deliquescent, and was stored in a silica gel desiccator. This reaction can more conveniently be carried out in a pressure reactor.

EXAMPLE 3

58.1 grams of dimethylaminopropylamine (0.569 moles) were added to a 250 ml flask, equipped as in Example 1, under nitrogen. 82 grams of caprylic acid (octanoic acid - 0.569 moles) (99%) were then added thereto. A small amount of heat was evolved, and the mixture was then heated to 180°C. The nitrogen sparge removed the water of reaction. After four hours at 180°C, the mixture was allowed to cool, to yield a viscous yellow/brown liquid with a base value of 243.7 (theoretical value 246.1) which is equivalent to a conversion of 99.0%.

EXAMPLE 4

Quarternization

70 grams of the product of Example 3 and 70 grams of methanol were added to a 250 ml flask, equipped as in Example 2. This mixture was then brought to reflux (at 70°C) and methyl chloride gas was passed into it for two and one-half hours. After cooling, the methanol was removed on a rotary film evaporator under vacuum at ~ 60°C. This yielded a pale yellow waxy solid with a base value of 3.7 (i.e. 98.5% conversion to quaternary).

EXAMPLE 5

(Preparation via ester route)

59 grams of ethyl lactate (0.5 moles) and 0.12 grams of sodium ethoxide were charged to a 250 ml flask fitted with a nitrogen sparge, thermometer, temperature controller and splash head take-off. 60 grams of dimethylaminopropylamine (0.59 moles) were then slowly added with nitrogen purging, with the mixture being heated to 120°C for two hours. Upon cooling, a light brown solid was formed with a base value of 320.8 (theoretical value 326.2), which indicates a conversion based on the base value of 97.2%. Infrared spectra were consistent with the formula:

$CH_3$-CH(OH)-CO-NH$(CH_3)_2$-N$(Me)_2$

EXAMPLE 6

Quaternization

75 grams of the product of Example 5 and 75 grams of methanol were charged to a 250 ml flask equipped as in Example 2. This mixture was then heated to reflux (70°C) and methyl chloride gas was passed therethrough for two and one-half hours. After cooling, the methanol was removed on a rotary film evaporator under vacuum at 60°C. This yielded a light brown crystalline solid with a base value of 3.4 (i.e. 99% conversion to quaternary).

EXAMPLES 7-14

By proceeding in the same manner as in Example 1 or 5, various compounds of the formula below were prepared:

R-CO-NH-$(CH_2)_3$-$N^+(Me)_3$ $Cl^-$;

EXAMPLE 7

R = H (using formic acid);

EXAMPLE 8

R = $CH_3CH_2$ - (using propionic acid);

EXAMPLE 9

R = $C_{11}H_{23}$* (using a coconut oil fatty acid mixture)

* (the chain length here represents an approximate average);

EXAMPLE 10

R = $C_{17}H_{33}$ oleic (octadecenoic) acid;

EXAMPLE 11
R = $C_{17}H_{35}$ stearic (octadecanoic) acid;

EXAMPLE 12
R = $C_{21}H_{43}$ behenic (docosanoic) acid;

EXAMPLE 13

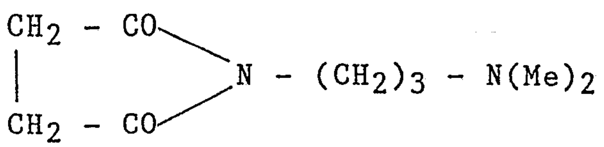

(using ethyl succinate and sodium ethoxide catalyst, as in Example 5);

EXAMPLE 14
$(Me)_2N-(CH_2)_3NH-CO-CO-NH-(CH_2)_3-N(Me)_2$
(using ethyl oxalate and sodium ethoxide catalyst, as in Example 5).

Each of these products was evaluated by allowing thin films in constant humidity to absorb moisture. The percent moisture absorbed was then calculated.

Approximately 2 to 5 grams of the compound being tested were placed in a pre-weighed Petri dish and oven dried to constant weight at 80° C. The dish was then placed in a constant humidity cabinet at 20° C and weighed half-hourly (approximately). The moisture uptake weight was then expressed as a percentage of the original weight.

Two relative humidities (R.H.) were used to evaluate the products:

1. 20% R.H. using potassium acetate as desiccant; and

2. 56.2% R.H. using potassium iodide as desiccant. The results obtained are set forth in Tables I and II hereof.

TABLE I

AT 20% R.H. % MOISTURE UPTAKE

(average of multiple determinations)

| Product of Example | TIME (HOURS) 0.5 | 1 | 1.5 | 2 | 2.5 | 3 | 3.5 | 4 | 4.5 | 5 | 5.5 | 6 |
|---|---|---|---|---|---|---|---|---|---|---|---|---|
| 1 | 1.0 | 2.6 | 4.0 | 5.9 | 7.75 | 9.21 | 10.62 | 12.05 | 13.2 | 14.41 | 15.75 | 17.1 |
| 3 | 1.0 | 1.8 | 2.9 | 3.4 | 3.8 | 4.2 | 4.8 | 5.1 | 5.9 | 6.4 | 7.3 | 8.9 |
| 5 | 0.9 | 1.1 | 2.3 | 6.7 | 7.3 | 8.35 | 9.0 | 10.0 | 10.55 | 11.15 | 11.7 | 12.4 |
| 7 | 3.0 | 13.0 | 21.55 | 28.8 | 23.6 | 24.3 | 24.9 | 25.4 | 25.85 | 26.3 | 28.6 | 26.9 |
| 8 | 0.3 | 0.5 | 1.4 | 1.9 | 2.4 | 2.9 | 3.3 | 3.75 | 4.0 | 4.4 | 9.7 | 5.3 |
| 9 | 0.6 | 1.05 | 1.7 | 2.3 | 2.75 | 3.1 | 3.35 | 3.65 | 3.9 | 4.2 | 4.4 | 4.7 |
| 10 | 0.5 | 0.75 | 1.1 | 1.25 | 1.4 | 1.55 | 1.6 | 1.75 | 1.9 | 2.0 | 2.1 | 2.2 |
| 11 | 0.6 | 1.15 | 1.7 | 2.3 | 2.4 | 2.45 | 2.6 | 2.75 | 2.8 | 2.9 | 2.9 | 2.9 |
| 12 | 0.4 | 0.8 | 1.6 | 2.05 | 2.2 | 2.7 | 2.9 | 3.15 | 3.3 | 3.65 | 3.8 | 3.9 |
| 13 | 1.2 | 2.45 | 3.0 | 4.8 | 6.55 | 8.0 | 9.8 | 11.0 | 12.9 | 13.9 | 14.6 | 16.3 |
| 14 | 0.4 | 0.8 | 1.2 | 1.65 | 1.8 | 2.1 | 2.5 | 2.9 | 3.15 | 3.3 | 3.3 | 3.3 |
| For comparison, the following commonly used humectants were also included | | | | | | | | | | | | |
| Glycerine | 0.6 | 1.3 | 1.5 | 1.85 | 1.9 | 2.0 | 2.3 | 2.8 | 3.15 | 3.6 | 3.9 | 4.3 |
| Acetic monoethanolamide | 0.7 | 1.8 | 2.3 | 2.8 | 3.4 | 3.85 | 4.3 | 4.85 | 5.4 | 5.95 | 6.5 | 7.2 |

TABLE II

AT 56.2% R.H. % MOISTURE UPTAKE

(average of multiple determinations)

| Product of Example | TIME (HOURS) | | | | | | | | | | | |
|---|---|---|---|---|---|---|---|---|---|---|---|---|
| | 0.5 | 1 | 1.5 | 2 | 2.5 | 3 | 3.5 | 4 | 4.5 | 5 | 5.5 | 6 |
| 1 | 15.2 | 17.85 | 20.0 | 22.3 | 23.9 | 24.1 | 26.2 | 28.35 | 31.0 | 32.95 | 33.6 | 36.5 |
| 3 | 4.25 | 7.9 | 10.0 | 12.05 | 16.25 | 19.25 | 19.75 | 20.3 | 21.0 | 21.9 | 22.8 | 24.0 |
| 5 | 0.6 | 2.85 | 5.85 | 7.0 | 9.45 | 11.3 | 12.95 | 15.0 | 16.2 | 16.85 | 18.3 | 19.0 |
| 7 | 12.1 | 21.4 | 29.3 | 33.1 | 34.6 | 37.9 | 38.9 | 40.4 | 42.05 | 42.9 | 43.8 | 44.3 |
| 8 | 2.15 | 4.15 | 6.25 | 8.15 | 11.0 | 12.25 | 14.35 | 16.7 | 17.15 | 18.05 | 20.0 | 20.8 |
| 11 | 0.8 | 1.4 | 2.3 | 3.1 | 4.4 | 5.35 | 6.1 | 7.05 | 8.1 | 8.35 | 8.6 | 8.7 |
| Glycerine | 0.4 | 1.8 | 2.8 | 3.95 | 5.15 | 6.80 | 7.4 | 8.25 | 9.0 | 9.9 | 10.0 | 10.8 |
| Acetic monoethanolamide | 0.4 | 1.9 | 3.0 | 4.1 | 5.3 | 6.9 | 7.65 | 8.45 | 9.25 | 10.0 | 11.0 | 12.8 |

EXAMPLE 15

403.2 grams (3.95 moles) of dimethylaminopropylamine were placed in a one litre, three-neck flask from which the air was displaced by nitrogen. 1.3 grams of 50% hypophosphorous acid was then added, and 262.8 grams (1.8 moles) of adipic acid was added, with mechanical stirring. The reaction mixture was exothermic to a temperature of about 115° C, and under distillation conditions was then heated with a slow nitrogen sparge to 185° C. The reaction mixture was then maintained at this temperature, with periodic additions of dimethylaminopropylamine (a total of 125 grams) until an acid value of less than 5 (4.49) and a base value in the range of 352-362 (354) was achieved. The charge was then cooled under nitrogen to 80° C. The substance produced was adip-bis (amidopropyldimethylamine).

EXAMPLE 16

574.8 grams (3.63 equiv.) of the amido-amine intermediate produced in Example 15, along with 240 grams of water, and 3 grams of sodium bicarbonate were placed in an autoclave from which the air was displaced by nitrogen. 185.3 grams (3.67 equiv.) of methyl chloride were then added at 75° C over a period of two hours, with a maximum pressure of 50 psi (350K pa). The reaction mixture was maintained at 75° C until a base value of less than 1 was obtained. Concentration of the resulting product was adjusted with water to obtain the following analysis: solids 75.3, free amine 0.45, amine hydrochloride 0.8, pH (3%) 4.3, colour (Gardner) 2, chloride 12.2. The product thus produced was adip-bis (amidopropyltrimethylammonium chloride).

EXAMPLE 17

314 grams (2 equiv.) of the amido-amine intermediate product in Example 15, along with 260 grams (1 equiv.) of cetyl chloride, and 63.8 grams of propylene glycol were placed in a one litre, three-neck flask from which the air was displaced by nitrogen. The flask was equipped with a mechanical stirrer-thermometer, nitrogen sparge, and reflux condenser, and was heated to 140° C. The reaction mixture was exothermic to a temperature of about 155° C. The reaction mixture was then cooled to 140° C, and maintained thereat for two hours, at which point the product had a base value of 89 (theoretical 88). 597 grams (.94 equiv.) of this material, along with 561.2 grams of water, and 3 grams of sodium bicarbonate were placed in an autoclave from which air was displaced by nitrogen. 46.8 grams (.93 equiv.) of methyl chloride was then added at 75° C over a period of two hours, with a maximum pressure of 50 psi. The reaction mixture was then maintained at 75-80° C and sampled for base value, after one hour, and hourly thereafter. Methyl chloride was added in 5 gram increments, until a base value less than 2 was obtained. The resulting product gave the following analysis: solids 52.7, active matter 49.2, chloride 5.7, amine hydrochloride 1.65, base value 1.9, pH (3%) 6.0, colour (Gardner) 3. The substance thus produced was adip-1-(amidopropyltrimethylammonium chloride)-6-(amidopropyl-dimethyl cetyl ammonium chloride).

Compounds of the present invention are water soluble humectants and in the following Examples, details of various compositions including the compounds are given. The specific compound used in the following formulations is either acetamidopropyltrimethyl ammonium chloride, INCROMECTANT AQ, the product of Example 2 or lactamidopropyltrimethylammonium chloride, INCROMECTANT LQ, the product of Example 6.

EXAMPLE 18

A hair detangling conditioner was prepared incorporating the following (by wt %):

| | | |
|---|---|---|
| INCROMECTANT LQ | (Lactamidopropyltrimethylammonium chloride) | 0.75 |
| INGROQUAT BA-85 | (Babasoamidopropalkonium chloride) | 1.00 |
| INCROMATE ISML | (Isostearamidopropyl morpholine lactate) | 1.00 |
| INCROQUAT BEHENYL TMS | (Behenyl trimethylammonium methosulfate (and) cetostearyl alcohol) | 0.25 |
| LANEOXOL AWS | (PPG-12-PEG-50 Lanolin) | 2.00 |
| CRODAPEARL LIQUID | (Sodium Laureth sulfate (and) hydroxyethylstearamide MIPA) | 3.00 |
| Germaben II | | 1.00 |
| Water, distilled | | 91.00 |

The above ingredients, with the exception of CRODAPEARL and some of the water were blended and heated to 60-65°C. Once the mixture was uniform, the mixture was cooled to 40°C with mixing and the CRODAPEARL added together with the remainder of the water in the form of a citric acid solution, whereby the pH was brought to 6.6 ± 0.8.

The resulting composition can be applied as a leave in or rinse out conditioner without any buildup or flaking.

EXAMPLE 19

A moisturising mist was prepared incorporating the following (by wt %):

| | | |
|---|---|---|
| INCROMECTANT AQ | (Acetamidopropyl trimethylammonium chloride) | 2.50 |
| CROQUAT WKP | (Cocodimonium hydrolysed animal keratin) | 1.00 |
| INCROPOL CS-50 | (Cetoareth-50) | 1.00 |
| FLUILAN AWS | (PPG-12-PEG-65 Lanolin Oil) | 2.00 |
| Propylene Glycol | | 7.00 |
| Germall II | | 1.00 |
| Water, deionised | | 85.50 |

The above ingredients, with the exception of CROQUAT WKP were mixed and heated to 70-75°C until thoroughly mixed. It was then cooled to room temperature with mixing and the CROQUAT added and the pH adjusted to 4.9.

The resulting composition acts as an aid in sealing the required level of moisture in the hair after a permanent wave process. The INCROMECTANT AQ's moisturisation properties allow for easy styling and manageability.

EXAMPLE 20

A clear collagen facial conditioner was prepared incorporating the following (by wt %)

| | | |
|---|---|---|
| Deionised Water | | 89.00 |
| CLEARCOL | (Soluble Animal Collagen) | 5.00 |
| RETICUSOL | (Soluble Reticulin) | 1.00 |
| CROTEIN CAA/SF | (Animal Collagen Amino Acids) | 1.00 |
| INCROMEC-TANT AQ | (Acetamidop-ropyl trimethylam-monium chloride) | 1.50 |
| PVP K-90 | | 1.00 |
| CMC-7MF | | .50 |
| Germaben II | | 1.00 |

The CLEARCOL was first dissolved in water and heated to no more than 30°C. The PVP-K90 and CMC-7MF were added. The remaining ingredients were mixed in and the pH adjusted to 6.5 by addition of 10% TEA. A clear colourless solution resulted which was found useful as a facial conditioner to tighten and moisturise the skin.

EXAMPLE 21

A household product was prepared by incoporating 0.5 wt% INCROMECTANT AQ in a 4% INCROSOFT T-90 dilution.

The product had a marked increase in its antistatic properties.

**Claims**

1. A compound of the formula:

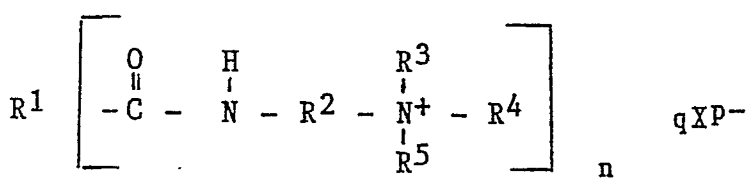

(I)

or

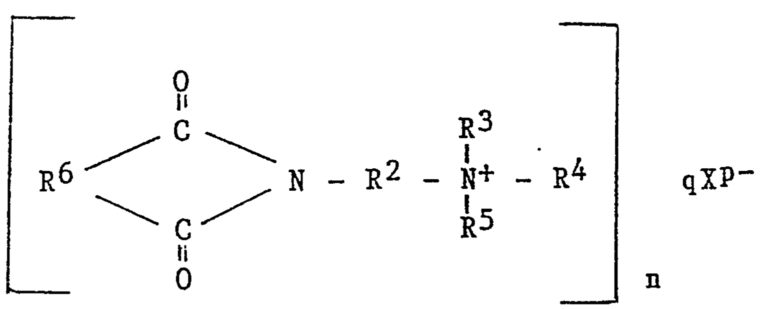

(II)

and mixtures of any two or more thereof, wherein n is 1 or 2 and q and p are positive integers, with the

product of q and p being equal to n; $R^1$ is a saturated or unsaturated, substituted or unsubstituted hydrocarbyl radical, and when n is 1, $R^1$ may be hydrogen, and when n is 2, $R^1$ may be a single bond; $R^2$ is a substituted or unsubstituted alkylene group, $R^3$, $R^4$ and $R^5$ are independently selected from substituted and unsubstituted alkyl groups; and $R^6$ is an ethylene group, a substituted ethylene group, or an ortho-aromatic structure.

2. A compound according to claim 1, wherein $R^2$ is a substituted or unsubstituted propylene group.

3. A compound according to claim 1 or 2, wherein $R^3$, $R^4$ and $R^5$ are independently selected from substituted and unsubstituted methyl groups.

4. A compound according to claim 1, 2 or 3, wherein $R^1$ is a hydroxy-substituted hydrocarbyl group.

5. A method of making a humectant compound as claimed in claim 1, which comprises reacting a carboxylic acid or derivative thereof with an N, N-disubstituted alkyldiamine to form the corresponding amide, and quaternizing said amide with an alkylating agent.

6. A method according to claim 5, wherein said N, N-disubstituted alkyldiamine is a compound of the formula:

$$H_2N - R^7 - \underset{}{\overset{\overset{\displaystyle R^8}{|}}{N}} - R^9$$

wherein $R^7$ is a substituted or unsubstituted alkylene group, and $R^8$ and $R^9$ are independently selected from substituted and unsubstituted alkyl groups.

7. A method according to claim 5 or 6, wherein a monocarboxylic acid is used selected from formic acid, acetic acid, propionic acid, lactic acid, butyric acid, pentanoic acid, undecanoic acid, dedecanoic acid, tridecanoic acid, tetradecanoic acid, pentadecanoic acid, hexadecanoic acid, heptadecanoic acid, octadecanoic acid, nonadecanoic acid, eicosanoic acid, docosanoic acid, tetracosanoic acid, and mixtures thereof; or a di-carboxylic acid is used selected from oxalic acid, malonic acid, glutaric acid, adipic acid, pimelic acid, sebacic acid, succinic acid, and mixtures thereof.

8. A method according to claim 5 or 6, wherein the carboxylic acid is an unsaturated mono- or dicarboxylic acid selected from acrylic acid, octadecenoic acid, docosenoic acid, and mixtures thereof; and fumaric acid.

9. A cosmetic composition, particularly for the care, protection or cleansing of the skin, which comprises up to 30% by weight of one or more compounds of claim 1, the balance being carrier and other ingredients such as emulsifiers, fats, vegetable extracts, preservatives, perfumes, thickeners, solvents and surfactants.

10. The use as a humectant of a compound as claimed in any of claims 1 to 4.